(19) Europäisches Patentamt
European Patent Office
Office européen des brevets

(11) Veröffentlichungsnummer: **0 007 406**
**A1**

# (12) EUROPÄISCHE PATENTANMELDUNG

(21) Anmeldenummer: 79101816.1

(22) Anmeldetag: 08.06.79

(51) Int. Cl.³: C 07 D 233/50
C 07 D 413/12, A 61 K 31/415

(30) Priorität: 10.07.78 DE 2830278

(43) Veröffentlichungstag der Anmeldung: 06.02.80 Patentblatt 80/3

(84) Benannte Vertragsstaaten: BE CH DE FR GB IT LU NL SE

(71) Anmelder: C.H. BOEHRINGER SOHN
Postfach 200
D-6507 Ingelheim am Rhein(DE)

(72) Erfinder: Stähle, Helmut, Dr.
Rotweinstrasse 23
D-6507 Ingelheim/Rhein(DE)

(72) Erfinder: Köppe, Herbert, Dr.
Neuweg 72
D-6507 Ingelheim/Rhein(DE)

(72) Erfinder: Kummer, Werner, Dr.
Georg-Scheuing-Strasse 15
D-6507 Ingelheim/Rhein(DE)

(72) Erfinder: Kobinger, Walter, Prof. Dr.
Belghofergasse 27
A-1120 Wien(AT)

(72) Erfinder: Lillie, Christian, Dr.Mag.
Hansi-Niese-Weg 12
A-1130 Wien(AT)

(72) Erfinder: Pichler, Ludwig, Dr.
Gusshausstrasse 24
A-1040 Wien(AT)

## (54) Neue substituierte 2-Phenylamino-imidazoline-(2), deren Säureadditionssalze, diese enthaltende Arzneimittel und Verfahren zur Herstellung derselben.

(57) Die Erfindung betrifft substituierte 2-(Phenylamino)-imidazoline-(2) der allgemeinen Formel

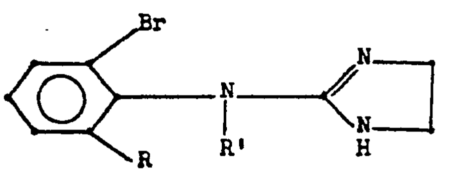

I

worin R' den Rest $-CH_3$, $-C_2H_5$, $-n-C_3H_7$, $i-C_3H_7$, $n-C_4H_9$, $-CH_2-CH(CH_3)_2$, $-n-C_5H_{11}$, $-(CH_2)_2-CH(CH_3)_2$, $-CH_2-C(CH_3)=CH_2$, $-CH_2-CH=CH-CH_3$, $-(CH_2)_2-CH=CH_2$, $-(CH_2)_2-C(CH_3)=CH_2$, $-CH_2-CH=C(CH_3)_2$, $-CH_2-CH=CH-C_6H_5$, $-CH_2-C_6H_5$, -cyclo-$C_5H_9$, -cyclo-$C_6H_{11}$, $-O-CH_2-$ , $-(CH_2)_2-$, $-(CH_2)_2-N(C_2H_5)_2$ und R Brom oder Fluor bedeuten sowie deren Säureadditionssalze.

Die neuen Verbindungen sind als Herz- bzw. Coronartherapeutika verwendbar.

EP 0 007 406 A1

BEZEICHNUNG
siehe Titelseite

Die Erfindung betrifft neue substituierte 2-Phenylamino-imidazoline-(2) der allgemeinen Formel

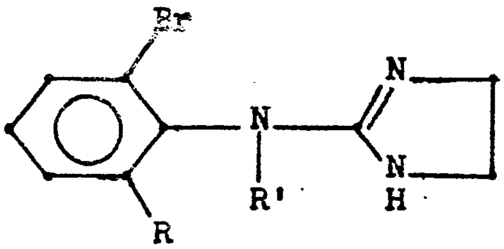

I

sowie deren physiologisch verträgliche Säureadditionssalze mit wertvollen therapeutischen Eigenschaften.

In der Formel I bedeutet R' den Rest $-CH_3$, $-C_2H_5$, $-n-C_3H_7$, $-i-C_3H_7$, $-n-C_4H_9$, $-CH_2-CH(CH_3)_2$, $-n-C_5H_{11}$, $-(CH_2)_2-CH(CH_3)_2$, $-CH_2-C(CH_3)=CH_2$, $-CH_2-CH=CH-CH_3$, $-(CH_2)_2-CH=CH_2$, $-(CH_2)_2-C(CH_3)=CH_2$ $-CH_2-CH=C(CH_3)_2$, $-CH_2-CH=CH-C_6H_5$, $-CH_2-C_6H_5$, -cyclo-$C_5H_9$, -cyclo-$C_6H_{11}$, $-O-CH_2-$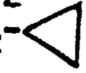, $-(CH_2)_2-$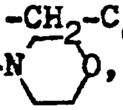, $-(CH_2)_2-N(C_2H_5)_2$ und R Brom oder Fluor.

Die Herstellung der Verbindungen der Formel I erfolgt durch:

a) Umsetzung eines 2-Phenylimino-imidazolidins der allgemeinen Formel

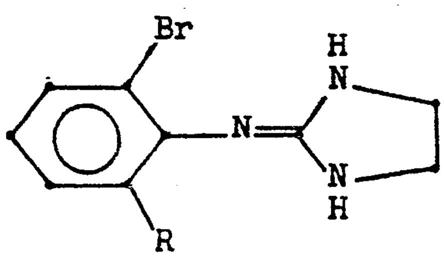

II

in der R die oben genannten Bedeutungen besitzt, mit einem Halogenid der allgemeinen Formel

Hal - R' III

worin Hal ein Chlor-, Brom-oder Jodatom bedeutet und R' die obige Bedeutung hat; oder

b) Umsetzung einer Verbindung der allgemeinen Formel

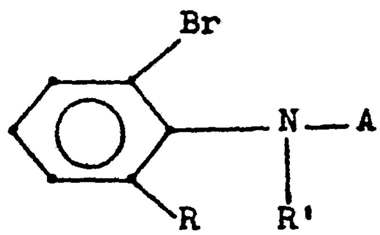

IV

in der R und R' wie oben angegeben definiert sind und

A eine Cyanogruppe oder den Rest 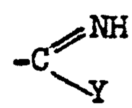 bedeutet, wobei Y eine Alkoxy- oder Alkylthiogruppe mit bis zu 4 C-Atomen oder eine Sulfhydril- oder Aminogruppe darstellt, mit Äthylendiamin oder dessen Säureadditionssalzen.

c) zur Herstellung von 2-[N-(substituiertes-Phenyl)-N-(cyclopropyl-methoxy)-amino]-imidazolinen-(2) der allgemeinen Formel

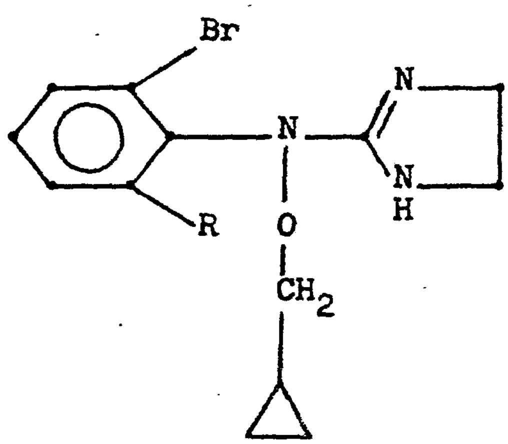

V

worin R die obige Bedeutung hat, setzt man ein 2-[N-(substituiertes-Phenyl)-N-hydroxyamino]-imidazolin-(2) der allgemeinen Formel

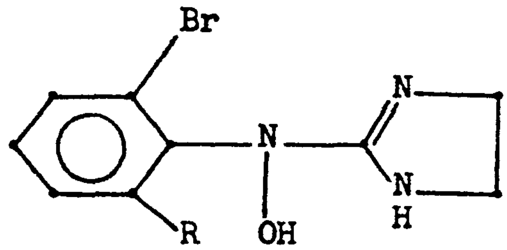

VI

worin R wie oben angegeben definiert ist, mit einem Cyclopropylmethylhalogenid der allgemeinen Formel

$Hal-CH_2-$ 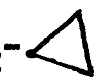

VII

worin Hal die obige Bedeutung hat, um.

Bei der Alkylierung der 2-Arylimino-imidazolidine der Formel II nach Verfahren a) erfolgt die Substitution ausschließlich am Brückenstickstoffatom. Bei der Umsetzung nach den Verfahren b) und c) ist die Konstitution der Endverbindungen durch die Synthese festgelegt. Die Position der Substituenten läßt sich außer durch die Synthese auch durch die NMR-Spaktroskopie festlegen. (Vgl. H. Stähle und K.-H. Pook, Liebigs Ann. Chem. 751, 159 ff (1971).

Die Umsetzung nach Verfahren a) und c) erfolgt zweckmäßigerweise durch Erhitzen der Reaktionspartner - vorzugsweise in Gegenwart eines polaren oder unpolaren organischen Lösungsmittels - auf Temperaturen von etwa 50 bis 150°C. Die speziellen Reaktionsbedingungen hängen in starkem Maße von der Reaktivität der Umsetzungsteilnehmer ab. Es empfiehlt sich, bei der Alkylierung des Halogenid im Überschuß anzuwenden und die Umsetzung in Gegenwart eines säurebindenden Mittels durchzuführen.

Bei dem Verfahren nach b) ist es erforderlich, bei erhöhter Temperatur, zwischen 60°C und 180°C zu arbeiten. Lösungsmittel sind nicht erforderlich. Es ist zweckmäßig, das als Reaktionspartner verwendete Äthylendiamin bzw. dessen Säureadditionssalz, im Überschuß anzuwenden.

Ausgangsverbindungen der Formel II sind z.B. in den belgischen Patenten Nr. 623 305, 687 657 und 705 944 beschrieben.

Ausgangsverbindungen der FormelnIII und VII lassen sich durch Halogenierung der zugrundeliegenden primären Alkohole darstellen.

Verbindungen der Formel IV erhält man ausgehend von Anilinen, durch Umsetzung mit Verbindungen der Formel III und nachfolgende Reaktion der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen gelangt man zu Cyanamiden, an die Ammoniak unter Bildung von Guanidinen angelagert werden kann.

Die Ausgangsverbindungen der Formel VI erhält man durch Oxydation von Verbindungen der allgemeinen Formel II mit Persäuren entsprechend DE-OS 24 57 979.

Die erfindungsgemäßen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propio-

säure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie intakten, narkotisierten Ratten untersucht.

Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

Beispiel 1

2-[N-(2,6-Dibromphenyl)-N-(n-propyl)amino]-2-imidazolin

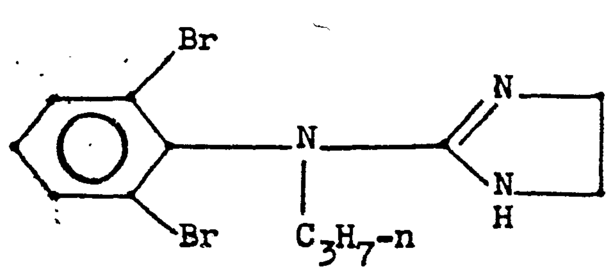

7,3 g (0,023 Mol) 2-(2,6-Dibromphenylimino)imidazolidin werde zusammen mit 2,3 ml (110 %) n-Propylbromid und 6,0 ml Triäthylamin in 30 ml absolutem Toluol 3 Tage lang im Rohr bei 110°C erhitzt. Die Reaktionsmischung wird nach dem Erkalten abdekantiert. Es hinterbleibt ein Rückstand, der in verdünnte Salzsäure aufgenommen und mehrmals mit Äther ausgeschüttelt wird (Ätherextrakte werden verworfen). Hierauf wird bei aufsteigenden pH-Werten (Alkalisieren mit verdünnter Natronlauge fraktioniert mit Äther extrahiert. Man erhält auf diese Weise ca. 10 Ätherextrakte. Diejenigen Fraktionen, welche die neue Verbindung in reiner Form enthalten (Kontrolle durch Dünnschi chromatogramm) werden vereinigt, über $CaSO_4$ getrocknet und de Äther im Vakuum abgezogen. Im Rückstand verbleiben 3,2 g 2-[N-(2,6-Dibromphenyl)-N-(n-propyl)-amino]-2-imidazolin (entsprechend einer Ausbeute von 37,2 % der Theorie). Schmelzpunkt: 155 - 156°C.

Dünnschichtchromatogramm:
Rf = 0,45
Fließmittelsystem:
Benzol : Dioxan : Äthanol : konz. Ammoniak = 50 : 40 : 5 : 5
Kieselgel-G-Fertigplatten Merck Nr. 60 F 254
Sprühreagens: Jodplatinat. Braune Flecken

<u>Beispiel 2</u>
2-[N-(2,6-Dibromphenyl)-N-(cyclopropylmethyloxy)-amino]-2-imidazolin.

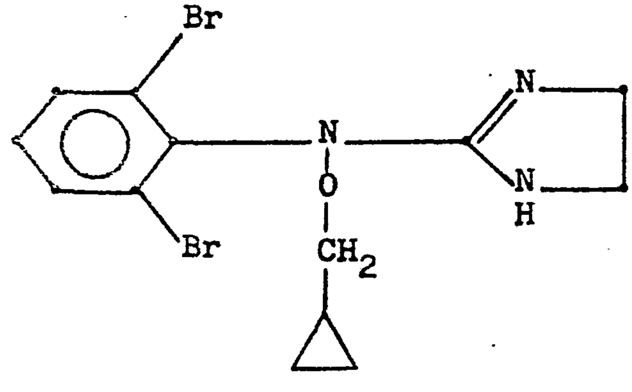

BAD ORIGINAL

6,0 g (0,016 Mol) 2-[N-(2,6-Dibromphenyl)-N-hydroxyamino]-2-imidazolin-hydrochlorid werden zu der Lösung aus 0,72 g Natrium in 75 ml absolutem Äthanol hinzugefügt. Sodann tropft man unter guter Rührung 1,7 g Chlormethylcyclopropan zu der Lösung hinzu und erwärmt 30 Minuten am Rückfluß. Das Lösungsmittel wird anschließend im Vakuum abgezogen und der verbleibende Rückstand in verdünnter Salzsäure gelöst. Nach mehrmaligem Ausschütteln der sauren, wässrigen Phase mit Äther (Ätherextrakte werden verworfen) bringt man die Lösung durch Zugabe von verdünnter Natronlauge auf pH 7. Die sich hierbei abscheidende Substanz (2-(2,6-Dibromphenylimino)imidazolidin) wird durch Absaugen entfernt und das Filtrat mit Natronlauge alkalisiert. Beim Aussalzen mit Kochsalz scheidet sich aus der alkalischen Mischung ein Niederschlag aus, der abgesaugt wird. Ausbeute: 0,6 g. Zur weiteren Reinigung wird das Substanzgemisch in verdünnter Salzsäure gelöst und die Lösung bei aufsteigenden pH-Werten (Alkalisieren mit verdünnter NaOH) so lange fraktioniert, mit Äther extrahiert, bis die letzte Verunreinigung entfernt ist. Sodannn wird die wässrige Phase alkalisiert. Hierbei scheidet sich die neue Imidazolinbase ab. Sie wird abgesaugt und getrocknet. Ausbeute: 0,1 g (1,5 % der Theorie)

Schmelzpunkt: 138 - 139°C.

Dünnschichtchromatogramm:

Rf = 0,9

Fließmittelsystem:

Essigester : Isopropanol : konz. Ammoniak = 70 : 50 : 20

Kieselgel-G-Fertigplatten Merck Nr. 60 F 254

Sprühreagens: Jodplatinat

(b) Rf = 0,1

Fließmittelsystem:

Methanol : Chloroform : Aceton = 60 : 150 : 30

Kieselgel-G-Fertigplatten Merck Nr. 60 F 254

Sprühreagens: Jodplatinat

**Beispiel 3**

2-[N-(1-Butenyl-(4))-N-(2,6-dibromphenyl)-amino]-2-imidazolin

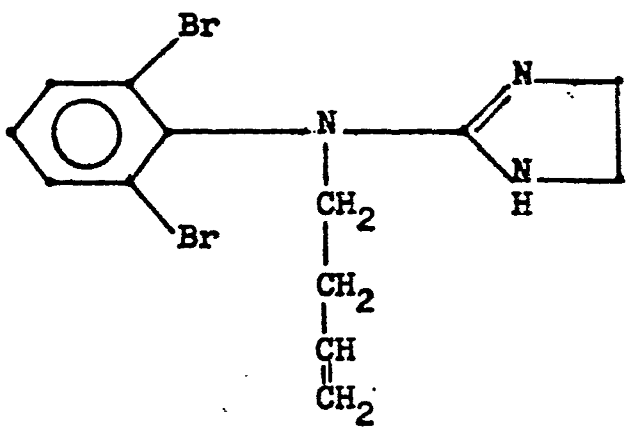

9,6 g (0,03 Mol) 2-(2,6-Dibromphenylimino)imidazolidin werden zusammen mit 5 ml 4-Brom-1-buten in 25 ml Methanol 16 Stunden lang bei 110°C im Rohr erhitzt. Die Reaktionsmischung wird hierauf im Vakuum zur Trockene eingeengt und der verbleibende Rückstand in verdünnter Salzsäure gelöst. Nach mehrmaligem Ausschütteln mit Äther (Ätherextrakte werden verworfen) wird bei aufsteigenden pH-Werten (stufenweises Alkalisieren mit verdünnter NaOH) fraktioniert mit Äther extrahiert (ca. 10 Fraktionen). Die dünnschichtchromatografisch einheitlichen Ätherextrakte werden vereinigt, über $MgSO_4$ getrocknet und der Äther im Vakuum abgezogen. Der Abdampfungsrückstand wird durch Anrühren mit wenig Petroläther zur Kristallisation gebracht. Ausbeute: 4,2 g (37,5 % der Theorie). Schmelzpunkt: 133 - 134°C.

Dünnschichtchromatogramm:

Rf = 0,4 (Kieselgel-G-Fertigplatten) Fließmittelsystem wie in Beispiel 1 angegeben.

**Beispiel 4**

2-[N-(Cinnamyl)-N-(2,6-dibromphenyl)-amino]-2-imidazolin-hydrobromid

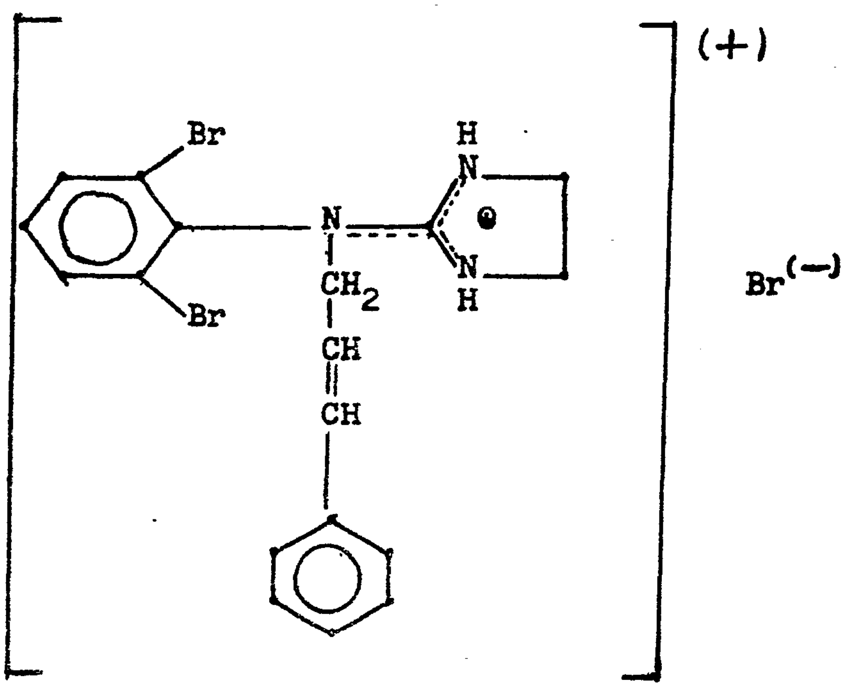

9,6 g (0,03 Mol) 2-(2,6-Dibromphenylimino)imidazolidin werden zusammen mit 6,5 g (110 %) Cinnamylbromid und 7 ml Triäthylamin in 50 ml absolutem Toluol 8 Stunden lang unter Rührung am Rückfluß erhitzt. Nach dem Erkalten wird die organische Phase abdekantiert und der Rückstand mit verdünnter Bromwasserstoffsäure behandelt (Ultraschallbad). Das entstandene Hydrobromid wird abgesaugt, mit Eiswasser gewaschen, zur weiteren Reinigung in Methanol gelöst und aus der Lösung durch Zugabe von Äther kristallisiert.
**Ausbeute:** 4,7 g (30,4 % der Theorie), Schmelzpunkt: 230 - 231°C.
$C_{18}H_{17}Br_2N_3$ x H Br (516,09)

| | C | H | Br | N |
|---|---|---|---|---|
| Ber.: | 41,89 | 3,52 | 46,45 | 8,14 |
| Gef.: | 41,96 | 3,43 | 46,00 | 8,14 |

Dünnschichtchromatogramm:
Rf = 0,75 (braune Flecken)
Fließmittelsystem:
Benzol : Dioxan : Äthanol : konz. Ammoniak = 50 : 40 : 5 : 5
Kieselgel-G-Fertigplatten Merck Nr. 60 F 254.
Sprühreagens: Jodplatinat

Analog den vorstehenden Beispielen wurden die folgenden Verbindungen hergestellt. Die Schmelzpunkte beziehen sich auf die Basen der Formel I. Anderenfalls ist die Salzform angegeben.

| Beispiel Nr. | R | R' | Fp. °C | Ausbeute % der Theo |
|---|---|---|---|---|
| 5 | Br | $-C_2H_5$ | 135-138 | 20,5 |
| 6 | Br | $-CH(CH_3)_2$ | 98-100 | 24,4 |
| 7 | Br | $-CH_2-C(CH_3)=CH_2$ | 120-122 | 41,1 |
| 8 | Br | $-CH_2-CH(CH_3)_2$ | 128-130 | 17,8 |
| 9 | Br | $-(CH_2)_2-$ 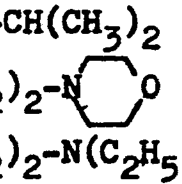 | 138-138,5 | 42,1 |
| 10 | Br | $-(CH_2)_2-N(C_2H_5)_2$ | 55-57 | 60,3 |
| 11 | Br | $n-C_4H_9$ | 136-138 | 26,7 |
| 12 | Br | $-cyclo-C_5H_9$ | 120-122 | 27,6 |
| 13 | Br | $-n-C_5H_{11}$ | 135-137 | 30,8 |
| 14 | Br | $-CH_3$ | 125-127 | 63,7 |
| 15 | Br | $-cyclo-C_6H_{11}$ | 147-149 | 4,0 |
| 16 | Br | $-(CH_2)_2-CH(CH_3)_2$ | 101-103 | 10,7 |
| 17 | Br | $-(CH_2)_2-C(CH_3)=CH_2$ | 126,5-127,5 | 34,0 |
| 18 | Br | $-CH_2-CH=CH-CH_3$ | 82-84 | 39,3 |
| 19 | Br | $-CH_2-CH=C(CH_3)_2$ | 187-188 (Hydrobromid) | 43,6 |
| 20 | Br | $-CH_2-C_6H_5$ | 87-91 | 57,9 |
| 21 | F | $-(CH_2)_2-CH(CH_3)_2$ | 106-107 | 48,5 |
| 22 | F | $-CH_2-CH=CH-CH_3$ | 102-102,5 | 49,5 |
| 23 | F | $-n-C_4H_9$ | 121 | 59,5 |
| 24 | F | $-CH_2-CH=C(CH_3)_2$ | Öl | 27,9 |
| 25 | F | $-n-C_5H_{11}$ | 98-99 | 49,9 |

Formulierungsbeispiele

Beispiel A: Dragées

| | | |
|---|---|---|
| Wirkstoff gemäß Erfindung | | 5 mg |
| Milchzucker | | 65 mg |
| Maisstärke | | 130 mg |
| sek. Calciumphosphat | | 40 mg |
| lösliche Stärke | | 3 mg |
| Magnesiumstearat | | 3 mg |
| kolloidale Kieselsäure | | 4 mg |
| | insgesamt | 250 mg |

Herstellung:
Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht gepreßt, die dann in üblicher Weise mit Hilfe von Zucker, Talkum und Gummi arabicum dragiert werden.

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:
Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

## PATENTANSPRÜCHE:

1. Substituierte 2-Phenylamino-imidazoline-(2) der allgemeinen Formel

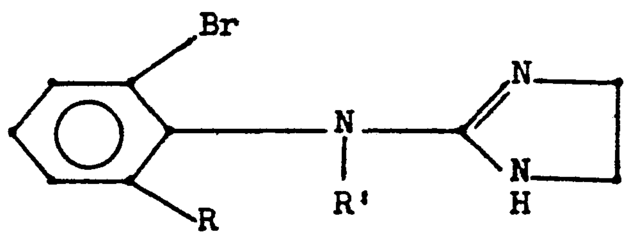

I

worin R' den Rest $-CH_3$, $-C_2H_5$, $-n-C_3H_7$, $-i-C_3H_7$, $n-C_4H_9$, $-CH_2-CH(CH_3)_2$, $-n-C_5H_{11}$, $-(CH_2)_2-CH(CH_3)_2$, $-CH_2-C(CH_3)=CH_2$, $-CH_2-CH=CH-CH_3$, $-(CH_2)_2-CH=CH_2$, $-(CH_2)_2-C(CH_3)=CH_2$, $-CH_2-CH=C(CH_3)_2$, $-CH_2-CH=CH-C_6H_5$, $-CH_2-C_6H_5$, -cyclo-$C_5H_9$, -cyclo-$C_6H_{11}$, $-O-CH_2-$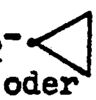, $-(CH_2)_2-$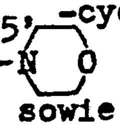, $-(CH_2)_2-N(C_2H_5)_2$ und R Brom oder Fluor bedeuten sowie deren Säureadditionssalze.

2. Verfahren zur Herstellung von Substituierten 2-Phenylaminoimidazolinen-(2) der allgemeinen Formel I nach Anspruch 1 und von deren Säureadditionssalzen, dadurch gekennzeichnet, daß man

a) ein 2-Phenyl-iminoimidazolidin der allgemeinen Formel

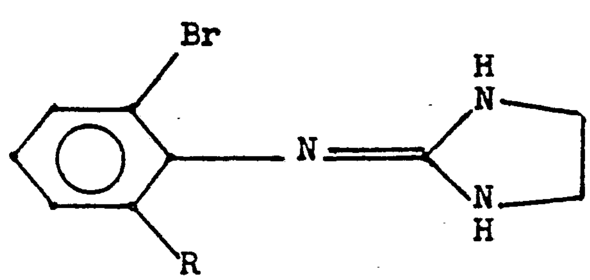

II

in der R die oben genannten Bedeutungen besitzt, mit einem Halogenid der allgemeinen Formel

Hal-R' III

worin Hal ein Chlor-, Brom- oder Jodatom bedeutet und R' die oben genannte Bedeutung hat, umsetzt oder

b) eine Verbindung der allgemeinen Formel

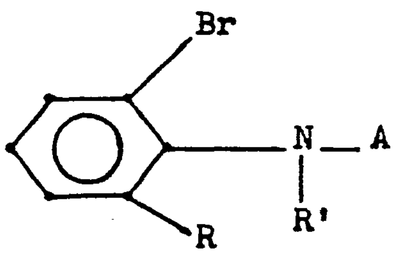

IV

in der R und R' wie oben angegeben definiert sind und A eine Cyanogruppe oder den Rest $-C\begin{smallmatrix} {=}NH \\ {-}Y \end{smallmatrix}$ bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt, oder

C) zur Herstellung von 2-[N-subst.-Phenyl)-N-cyclopropylmethoxyamino]-imidazolinen-(2) der allgemeinen Formel

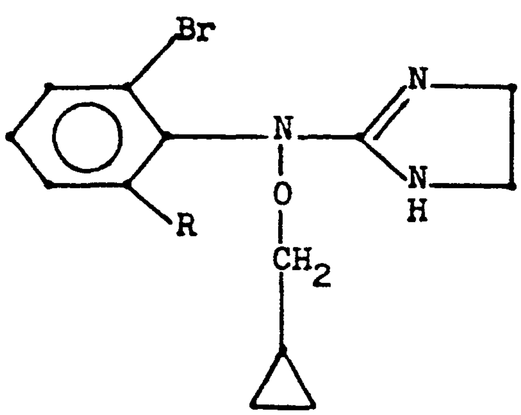

V

000740



worin R die obige Bedeutung hat, ein 2-[N-(subst.-Phenyl)-N-hydroxy-amino]-imidazolin-(2) der allgemeinen Formel

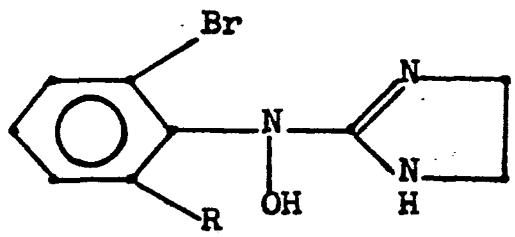

VI

worin R wie oben angegeben definiert ist, mit einem Cyclopropylmethylhalogenid der allgemeinen Formel

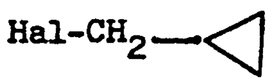

VII

worin Hal die obige Bedeutung hat, umsetzt und daß man gegebenenfalls das nach einem der Verfahren erhaltene Endprodukt in ein Säureadditionssalze überführt.

3. Verfahren nach Anspruch 2, dadurch gekennzeichnet, daß man die Umsetzung bei Temperaturen von etwa 50 bis 100°C durchführt.

4. Verfahren nach Anspruch 2 a) und c) und/oder 4, dadurch gekennzeichnet, daß man die Umsetzung in Gegenwart eines polaren oder unpolaren organischen Lösungsmittels durchführt.

5. Verfahren nach Anspruch 2 a) und c) bis 5, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff eine oder mehrere Verbindungen der allgemeinen Formel I oder deren Säureadditionssalze enthalten.

7. Verfahren zur Herstellung von Arzneimittels nach Anspruch 6,

dadurch gekennzeichnet, daß man eine oder mehrere Verbindungen der allgemeinen Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verwendung von Verbindungen der allgemeinen Formel I oder deren physiologisch verträglichen Säureadditionssalzen bei der Bekämpfung von Herz- und Coronarerkrankungen.

0007406

Europäisches Patentamt

# EUROPÄISCHER RECHERCHENBERICHT

Nummer der Anmeldung

EP 79 101 816.1

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | DE - A - 1 957 722 (C.H. BOEHRINGER SOHN)<br>* Ansprüche 1, 4, 7 bis 9, 15 *<br>-- | 1-8 | C 07 D 233/50<br>C 07 D 413/12<br>A 61 K 31/415 |
| | DE - A - 1 958 212 (C.H. BOEHRINGER SOHN)<br>* Ansprüche 1, 4, 7 bis 10 *<br>-- | 1-7 | |
| | DE - A - 2 208 434 (C.H. BOEHRINGER SOHN)<br>* Ansprüche 1 bis 4, 6, 7 *<br>-- | 1-4, 6,7 | RECHERCHIERTE SACHGEBIETE (Int. Cl.3)<br>A 61 K 31/415<br>C 07 D 233/50<br>C 07 D 413/12 |
| | DE - A - 2 259 160 (C.H. BOEHRINGER SOHN)<br>* Ansprüche 1 bis 4 *<br>---- | 1,2, 6,7 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 06-11-1979 | FROELICH |